Europäisches Patentamt

(19) European Patent Office

Office européen des brevets

(11) **EP 0 623 388 B1**

(12) **FASCICULE DE BREVET EUROPEEN**

(45) Date de publication et mention
de la délivrance du brevet:
**02.12.1998 Bulletin 1998/49**

(51) Int. Cl.$^6$: **B01J 31/02**, B01J 27/053,
C07C 2/62

(21) Numéro de dépôt: **94400776.4**

(22) Date de dépôt: **11.04.1994**

(54) **Catalyseur d'alkylation de paraffines**

Katalysator für die Alkylierung von Paraffinen

Catalyst for the alkylation of paraffins

(84) Etats contractants désignés:
**BE DE FR GB IT NL SE**

(30) Priorité: **20.04.1993 FR 9304735**

(43) Date de publication de la demande:
**09.11.1994 Bulletin 1994/45**

(73) Titulaire:
**INSTITUT FRANCAIS DU PETROLE
92502 Rueil-Malmaison (FR)**

(72) Inventeurs:
• **Joly, Jean-François
F-75003 Paris (FR)**
• **Ferrer, Nathalie
F-78400 Chatou (FR)**
• **Bernhard, Jean-Yves
F-91540 Mennecy (FR)**
• **Benazzi, Eric
F-78360 Montesson (FR)**

(56) Documents cités:
EP-A- 0 433 954          FR-A- 2 682 891
FR-A- 2 683 739          US-A- 4 038 212

Printed by Xerox (UK) Business Services
2.16.6/3.4

## Description

La présente invention concerne un catalyseur à base d'un support poreux minéral ou organique, de préférence la silice, et du mélange constitué par l'acide sulfurique, l'acide trifluorométhanesulfonique et éventuellement l'eau, son mode de préparation et son utilisation en alkylation catalytique d'isobutane et/ou d'isopentane au moyen d'au moins une oléfine, qui permet d'obtenir au moins un produit dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et/ou isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine(s) liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires (voir par exemple US-A-3.251.902, US-A-3.647.916, US-A-4.377.721, US-A-4.384.161 et US-A-4.300.015), les résines macroréticu-laires éventuellement associées avec BF$_3$ (voir par exemple US-A-3.855.342, US-A-3.855.343, US-A-3.862.258 et US-A-3.879.489), les acides de Lewis et/ou de Brönsted déposés sur divers supports inorganiques (voir par exemple US-A-3.975.299, US-A-3.852.371 et US-A-3.979.476), les alumines chlorées (voir par exemple US-A-3.240.840, US-A-3.607.859, US-A-4.066.716, et US-A-4.083.800), les graphites intercalés par des acides de Lewis et/ou de Brönsted (voir par exemple US-A-4.083.885, US-A-4.116.880, US-A-4.128.596 et US-A-3.976.714) et les anions déposés sur supports oxydes tels que ZrO$_2$/SO$_4$ (voir par exemple J-01245854-A, J-01245853, et J-61242641-A). Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés), ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

La demande de brevet européen EP-A-0.433.954 revendique l'utilisation d'acides fluorosulfoniques, dont les acides FSO$_3$H et CF$_3$SO$_3$H, pour la réaction d'alkylation de l'isobutane par les oléfines en lit fixe. Les auteurs montrent, dans un exemple dudit brevet, que l'acide sulfurique mis en oeuvre dans les conditions selon ladite invention conduit à de très mauvais résultats.

La demande de brevet européen EP-A-0.539.277 décrit un catalyseur comprenant de la silice et de l'acide sulfurique à l'état solide, la silice ayant été imprégnée par une solution comprenant de l'acide sulfurique et éventuellement un additif dont l'acide trifluorométhanesulfonique CF$_3$SO$_3$H.

EP-A-0 605 279 décrit un catalyseur comprenant un support poreux organique ou minéral, de préférence de la silice, et le mélange équimolaire constitué par les acides trifluorométhanesulfonique et sulfurique anhydres.

Dans la présente invention, on a découvert un nouveau catalyseur permettant d'obtenir des composés paraffiniques à hauts degrés de ramification et hauts indices d'octane par alkylation de l'isobutane et/ou de l'isopentane avec au moins une oléfine comprenant de 3 à 6 atomes de carbone par molécule. Ce nouveau catalyseur est avantageusement mis en oeuvre dans le réacteur en phase liquide et en mélange avec l'isoparaffine et/ou un mélange d'isoparaffines. Le catalyseur selon l'invention peut être mis en oeuvre en lit expansé, en réacteur parfaitement agité ou en lit circulant.

Le catalyseur de la présente invention renferme un support poreux organique ou minéral, de préférence la silice, et un mélange constitué par l'acide sulfurique, l'acide trifluorométhanesulfonique et éventuellement l'eau, le support ayant été imprégné partiellement ou totalement par ledit mélange.

La composition pondérale dudit mélange est comprise dans les intervalles de valeurs suivants :

- acide sulfurique : entre 80 et 99,5 %, de préférence entre 85 et 99,5 %,

- acide trifluorométhanesulfonique : entre 0,5 et 15 %, de préférence entre 0,8 et 15 %,
- eau : entre 0 et 5 %, de préférence entre 0,05 et 3 %.

Dans le cas où la silice est utilisée comme support, elle peut contenir des impuretés comme par exemple les oxydes, les alcalins, les alcalino-terreux, les composés d'aluminium ou toute autre impureté connue de l'homme du métier, la quantité totale de ces impuretés n'excédant pas 2 % en poids par rapport à la silice. De nombreuses sources de silice peuvent être utilisées. La surface spécifique du support poreux organique ou minéral doit être comprise entre 0,01 et 1500 $m^2$/g, de préférence entre 0,01 et 150 $m^2$/g. Le volume poreux total dudit support doit être compris entre 0,005 et 3 $cm^3$/g, de préférence entre 0,005 et 1,5 $cm^3$/g et de manière souvent plus préférée entre 0,005 et 0,8 $cm^3$/g. Ledit support est constitué de grains sensiblement sphériques de diamètres compris entre 80 et 150 $\mu$m.

Lors de l'imprégnation du support poreux minéral ou organique, le mélange constitué par l'acide sulfurique, l'acide trifluorométhanesulfonique et l'eau doit occuper un fraction du volume poreux total comprise entre 5 et 100 %, de préférence entre 80 et 100 %, et de manière encore plus préférée entre 90 et 100 %. Le catalyseur ainsi obtenu est caractérisé par une surface spécifique comprise entre 0,01 et 500 $m^2$/g, de préférence entre 0,01 et 150 $m^2$/g et de manière encore plus préférée entre 0,01 et 40 $m^2$/g.

Le procédé de préparation préféré du catalyseur selon l'invention comprend les trois étapes suivantes, l'ordre des deux premières étapes pouvant être inversé :

- Dans une première étape, le mélange constitué par l'acide sulfurique, l'acide trifluorométhanesulfonique et éventuellement de l'eau, est préparé par exemple par addition lente et sous agitation d'acide trifluorométhanesulfonique dans la totalité d'une solution comprenant la totalité de l'acide sulfurique et éventuellement de l'eau. La durée totale d'injection de l'acide trifluorométhanesulfonique est habituellement comprise entre quelques minutes et 1 heure. La préparation de ce mélange ainsi que sa conservation, doivent être effectuées à l'abri de l'humidité.

- Dans une seconde étape le support poreux organique ou minéral est calciné à une température généralement supérieure à 50 °C, de préférence supérieure à 80 °C et de manière encore plus préférée comprise entre 200 et 600 °C, par exemple égale à environ 500 °C. La durée de cette étape de calcination est habituellement comprise entre 10 minutes et 50 heures. La calcination peut être effectuée en présence d'air ou de mélange air/azote, de débit compris entre 0,001 et 10 l/h/g.

- La troisième étape consiste en l'imprégnation du support poreux organique ou minéral calciné par le mélange constitué par l'acide sulfurique, l'acide trifluorométhanesulfonique et éventuellement l'eau. Pour réaliser cette étape on peut utiliser toutes les techniques connues de l'homme du métier. Conservé à l'abri de l'humidité, le catalyseur selon la présente invention est ainsi constitué d'un support poreux organique ou minéral imprégné par le mélange constitué par l'acide sulfurique, l'acide trifluorométhane-sulfonique et éventuellement l'eau.

Le catalyseur est utilisé dans un procédé d'alkylation dans lequel on traite une charge comprenant d'une part au moins une isoparaffine, de préférence au moins un élément choisi dans le groupe formé par l'isobutane et l'isopentane, de manière encore plus préférée l'isobutane, et d'autre part au moins une oléfine contenant de 3 à 6 atomes de carbone par molécule, en présence du catalyseur constitué par un support poreux organique ou minéral imprégné par le mélange constitué par l'acide sulfurique, l'acide trifluorométhanesulfonique et éventuellement l'eau.

Le mélange isoparaffine(s)-oléfine(s) peut être introduit dans le réacteur à une vitesse spatiale horaire, exprimée en poids d'oléfine introduite par unité de poids du catalyseur et par heure comprise entre 0,001 et 10 $h^{-1}$ et de préférence comprise entre 0,002 et 2 $h^{-1}$. Ledit mélange peut aussi être réalisé à l'intérieur du réacteur. Dans tous les cas le mélange ainsi constitué est dans le réacteur dans des conditions de pression et de température telles que le mélange d'hydrocarbures reste liquide sur le catalyseur.

La température de réaction peut être comprise entre -50 et 150 °C, mais les performances catalytiques sont nettement améliorées lorsque la température de réaction est inférieure à 0 °C. La température de réaction est généralement inférieure à 0 °C, de préférence à -3 °C. La pression du réacteur est telle que les hydrocarbures sont maintenus à l'état liquide dans le réacteur.

Afin de limiter les réactions secondaires, on peut utiliser un excès d'isoparaffine(s) par rapport à l'(aux) oléfine(s). A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes dans la charge est généralement compris entre 1 et 100, de préférence entre 3 et 50 et de manière souvent préférée entre 5 et 10.

Les produits de la réaction peuvent être contrôlés régulièrement par mesure de l'indice de brome, par exemple selon le projet de Norme Française Pr. M. 07.071 de mars 1969.

Lorsque la nature du catalyseur et les conditions de travail du catalyseur sont judicieusement choisies (en particulier la température), le catalyseur selon l'invention permet la production de produits d'alkylation d'isoparaffine(s) par l'(les) oléfine(s) qui sont intéressants comme carburants pour les moteurs et constitutants d'essence. De préférence, les produits d'alkylation obtenus comprennent au moins 60 % mole de paraffines possédant 8 atomes de carbone par molécule et moins de 1 % moles de composés non saturés, les paraffines possédant 8 atomes de carbone par molécule comprenant de 70 à 98 % (en moles) de triméthylpentane(s).

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

**Exemple 1 :** Préparation des catalyseurs.

*(a) Catalyseur conforme à l'invention : catalyseur A*

On active 16 g de silice macroporeuse de surface spécifique égale à 40 $m^2$/g, de volume poreux total égal à 1,2 $cm^3$/g, et constituée principalement de grains sensiblement sphériques de diamètre moyen égal à 110 $\mu$m, par calcination sous air pendant 4 heures à 500 °C. La silice ainsi activée est conservée sous argon. On procède alors à une imprégnation à sec, et à l'abri de l'humidité, de 14 g de ladite silice deshydratée par 22 g du mélange constitué par :

- 18,7 g d'une solution contenant 99 % poids d'$H_2SO_4$ et 1 % poids d'eau,
- 3,3 g d'une solution contenant 3,23 g d'acide $CF_3SO_3H$ et 0,07 g d'eau.

La composition pondérale de la phase acide est la suivante :

- $H_2SO_4$    : 84,15 %
- $CF_3SO_3H$  : 14,68 %
- $H_2O$      : 1,17 %

Le solide ainsi obtenu est conservé sous argon à -18 °C.

*(b) Catalyseur non conforme à l'invention : catalyseur B*

On prépare 14 g de silice deshydratée d'une façon identique à la préparation du catalyseur conforme à l'invention. On procède alors à un imprégnation à sec, et à l'abri de l'humidité, de 14 g de ladite silice par 22 g du mélange constitué par.

- 21,78 g d'$H_2SO_4$ et 0,22 g d'eau.

Le solide ainsi obtenu est conservé sous argon à -18 °C.

**Exemple 2** : Résultats des tests d'alkylation de l'isobutane par le butène-1.

Les catalyseurs A et B sont utilisés pour alkyler l'isobutane par le butène-1 de façon à produire des paraffines ramifiées à hauts indices d'octane. Les catalyseurs A et B sont testés selon le même protocole opératoire qui est décrit ci-après.

On introduit 36 g du catalyseur A ou B préparé dans l'exemple 1 dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

100 $cm^3$ d'isobutane sont alors ajoutés dans le réacteur contenant le catalyseur sous agitation, ledit réacteur étant immergé dans un bain froid à -6 °C. On laisse sous agitation le système catalyseur + isobutane pendant une durée de 30 minutes afin d'homogénéiser la température.

On ajoute en continu un mélange d'isobutane et de butène-1, contenant 20 % poids de butène-1, pendant une durée totale de 8 heures, la température du réacteur est maintenu à -5 °C pendant toute la durée de l'injection. Le débit volumique du butène-1 est égal à 10 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau ci-après. La conversion de l'oléfine est de 100 %.

|  | CATALYSEUR A | CATALYSEUR B |
|---|---|---|
| $C_5$-$C_7$ | 2,5 | 11,7 |
| $C_8$ total | 93 | 62,1 |
| $C_9^+$ | 4,5 | 26,2 |
| TMPs/$C_8$ | 94 | 87 |
| TMPs/$C_8$ : proportion de triméthylpentanes (isomères 224, 223, 234 et 233) dans la coupe $C_8$. | | |

Ce tableau met en évidence l'effet de la présence d'acide $CF_3SO_3H$ dans le catalyseur : à même débit de butène-1 on obtient une proportion de triméthylpentanes en utilisant le catalyseur A selon l'invention nettement supérieure par rapport à l'utilisation du catalyseur B de référence qui ne contient pas d'acide $CF_3SO_3H$. Le catalyseur A est plus actif et plus sélectif que le catalyseur de référence B.

## Revendications

1. Catalyseur comprenant un support poreux organique ou minéral et le mélange constitué par l'acide sulfurique, l'acide trifluorométhane-sulfonique et éventuellement l'eau, le support ayant été imprégné par ledit mélange et, avant imprégnation, ayant une surface spécifique comprise entre 0,01 et 1500 $m^2$/g, un volume poreux total compris entre 0,005 et 3 $cm^3$/g et étant principalement constitué de grains sensiblement sphériques de diamètre moyen compris entre 80 et 150 $\mu$m, et la composition pondérale dudit mélange étant comprise dans les intervalles de valeurs suivants :

   - acide sulfurique : entre 80 et 99,5 %,
   - acide trifluorométhanesulfonique : entre 0,5 et 15 %,
   - eau : entre 0 et 5 %.

2. Catalyseur selon la revendication 1 tel que ledit support est la silice.

3. Catalyseur selon l'une des revendications 1 ou 2 tel que ledit support, avant son imprégnation par ledit mélange, comprend au plus 2 % d'impureté.

4. Catalyseur selon l'une des revendications 1 à 3 tel que la composition pondérale dudit mélange est comprise dans les intervalles de valeurs suivants :

   - acide sulfurique : entre 85 et 99,5 %,
   - acide trifluorométhanesulfonique : entre 0,8 et 15 %,
   - eau : entre 0,05 et 3 %.

5. Procédé de préparation d'un catalyseur selon l'une des revendications 1 à 4 tel que ledit support est calciné puis imprégné par le mélange constitué par l'acide sulfurique, l'acide trifluorométhanesulfonique et éventuellement l'eau.

6. Utilisation du catalyseur selon l'une des revendications 1 à 5 dans un procédé d'alkylation dans lequel on traite une charge comprenant d'une part au moins une isoparaffine choisie dans le groupe formé par l'isobutane et l'isopentane et d'autre part au moins une oléfine contenant de 3 à 6 atomes de carbone par molécule.

7. Utilisation selon la revendication 6 dans laquelle la température de la réaction est inférieure à 0 °C.

## Claims

1. Catalyst comprising a porous organic or mineral support and a mixture constituted by sulphuric acid, trifluoromethanesulphonic acid and, optionally, water, the support being impregnated by said mixture and, before impregnation, having a specific surface area of between 0.01 and 1500 $m^2/g$, a total pore volume of between 0.005 and 3 $cm^3/g$ and being principally constituted by substantially spherical particles having an average diameter of between 80 and 150 $\mu$m, said mixture having a composition by weight between the following limits:

- sulphuric acid: between 80 and 99.5 %,
- trifluoromethanesulphonic acid: between 0.5 and 15 %,
- water: between 0 and 5 %.

2. Catalyst according to claim 1 wherein said support is silica.

3. Catalyst according to claim 1 or claim 2 wherein said support, before impregnation with said mixture, contains at most 2 % of impurities.

4. Catalyst according to any one of claims 1 to 3 wherein the composition by weight of said mixture is between the following values:

- sulphuric acid: between 85 and 99.5 %,
- trifluoromethanesulphonic acid: between 0.8 and 15 %,
- water: between 0.05 and 3 %.

5. A process for the preparation of a catalyst according to any one of claims 1 to 4 wherein said support is calcined then impregnated with a mixture constituted by sulphuric acid, trifluoromethanesulphonic acid and, optionally, water.

6. Use of a catalyst in accordance with any one of claims 1 to 5 in an alkylation process which treats a feedstock comprising at least one isoparaffin selected from the group formed by isobutane and isopentane and at least one olefin containing 3 to 6 carbon atoms per molecule.

7. Use according to claim 6 wherein the reaction temperature is below 0°C.

**Patentansprüche**

1. Katalysator, umfassend einen porösen organischen oder mineralischen Träger sowie das Gemisch, bestehend aus Schwefelsäure, Trifluormethansulfonsäure und gegebenenfalls Wasser, wobei der Träger mit diesem Gemisch imprägniert ist und vor der Imprägnierung eine spezifische Oberfläche zwischen 0,01 und 1500 $m^2/g$, ein Porengesamtvolumen zwischen 0,005 und 3 $cm^3/g$ hat und hauptsächlich gebildet wird durch im wesentlichen kugelförmige Körner von einem mittleren Durchmesser zwischen 80 und 150 $\mu$m und die Gewichtszusammensetzung dieses Gemisches in den Intervallen folgender Werte liegt:

- Schwefelsäure: zwischen 80 und 99,5%,
- Trifluormethansulfonsäure: zwischen 0,5 und 15%, und
- Wasser: zwischen 0 und 5%.

2. Katalysator nach Anspruch 1, dadurch gekennzeichnet, daß der Träger Silicium(di)oxid ist.

3. Katalysator nach einem der Ansprüche 1 oder 2, derart, daß dieser Träger vor seiner Imprägnierung mit diesem Gemisch höchstens 2% Verunreinigungen umfaßt.

4. Katalysator nach einem der Ansprüche 1 bis 3, von der Art, daß die Gewichtszusammensetzung dieses Gemisches in den Intervallen folgender Werte liegt:

- Schwefelsäure: zwischen 85 und 99,5%,
- Trifluormethansulfonsäure: zwischen 0,8 und 15%, und
- Wasser: zwischen 0,05 und 3%.

5. Verfahren zur Herstellung eines Katalysators gemäß einem der Ansprüche 1 bis 4 von der Art, daß dieser Träger calciniert, dann durch das durch Schwefelsäure, Trifluormethansulfonsäure und gegebenenfalls Wasser gebildete Gemisch imprägniert wird.

**6.** Verwendung des Katalysators nach einem der Ansprüche 1 bis 5 bei einem Alkylierungsverfahren, bei dem man eine Charge behandelt, die einerseits wenigstens ein Isoparaffin, gewählt aus der durch Isobutan und Isopentan gebildeten Gruppe und andererseits wenigstens ein Olefin umfaßt, das 3 bis 6 Kohlenstoffatome pro Molekül enthält.

**7.** Verwendung nach Anspruch 6, bei dem die Temperatur der Reaktion unterhalb 0°C liegt.